# EUROPÄISCHE PATENTSCHRIFT

(11) **EP 1 444 244 B1**
(45) Veröffentlichungstag und Bekanntmachung des Hinweises auf die Patenterteilung: **29.06.2005**
(21) Anmeldenummer: 02802638.3
(22) Anmeldetag: 02.11.2002
(51) Int. Cl.: C07H 17/08

(54) **VERFAHREN ZUR HERSTELLUNG VON DESCLARITHROMYCIN, SOWIE ZWISCHENPRODUKTE**
METHOD FOR THE PRODUCTION OF DESCLARITHROMYCIN, AND INTERMEDIATE PRODUCTS
PROCEDE DE FABRICATION DE DESCLARITHROMYCINE ET PRODUITS INTERMEDIAIRES

(30) Priorität: 07.11.2001 DE 10154244; 21.12.2001 DE 10163550; 05.01.2002 DE 10200252
(43) Veröffentlichungstag der Anmeldung: 11.08.2004
(73) Patentinhaber: Aventis Pharma Deutschland GmbH, 65929 Frankfurt am Main (DE)
(72) Erfinder: JENDRALLA, Heiner, 65931 Frankfurt (DE); KORB, Gerhard, 63512 Hainburg (DE); MUELLER-LEHAR, Jürgen, 55278 Weinolsheim (DE)
(86) Internationale Anmeldenummer: PCT/EP2002/012234
(87) Internationale Veröffentlichungsnummer: WO 2003/040162

(56) Entgegenhaltungen:
- WO-A-97/36912
- WO-A-97/38000
- C.SHENGXI: "3-Keot-9-O-substituted Oxime Derivatives of 6-O-Methyl Erythromycin A Synthesis and In Vitro Activity." JOURNAL OF ANTIBIOTICS, Bd. 54, Nr. 6, 2001, Seiten 506-509, XP001109508
- AGOURIDAS C ET AL: "SYNTHESIS AND ANTIBACTERIAL ACTIVITY OF KETOLIDES (6-O-METHYL-3-OXOERYTHROMYCIN DERIVATIVES): A NEW CLASS OF ANTIBACTERIALS HIGHLY POTENT AGAINST MACROLIDE-RESISTANT AND -SUSCEPTIBLE RESPIRATORY PATHOGENS" JOURNAL OF MEDICINAL CHEMISTRY, AMERICAN CHEMICAL SOCIETY. WASHINGTON, US, Bd. 21, Nr. 41, 8. Oktober 1998 (1998-10-08), Seiten 4080-4100, XP001068954 ISSN: 0022-2623

## Beschreibung

Die vorliegende Erfindung beschreibt ein Verfahren zur Herstellung von Desclarithromycin über die neuen Zwischenstufen des mit Silylgruppen geschützten Erythromycin N-oxids und 6-O-Methylerythromycin N-oxids. Außerdem wird die neue Zwischenstufe des Desclarithromycin N-oxids beschrieben.

Desclarithromycin (II) ist ein Baustein für neuartige Makrolidantibiotika. Die Herstellung von Desclarithromycin (II) ausgehend von Clarithromycin (I), das über verschiedene Synthesewege aus Erythromycin A (III) hergestellt werden kann, ist bekannt (siehe z. B. Abbott Laboratories WO 97/36912). Man gewinnt dabei das Desclarithromycin (II) durch saure Behandlung des Clarithromycins (I). Dabei wird selektiv die Cladinose abgespalten und das resultierende Desclarithromycin (II) gewonnen (J. Med. Chem. 1998, 41, 4080 - 4100) (Formelschema I)

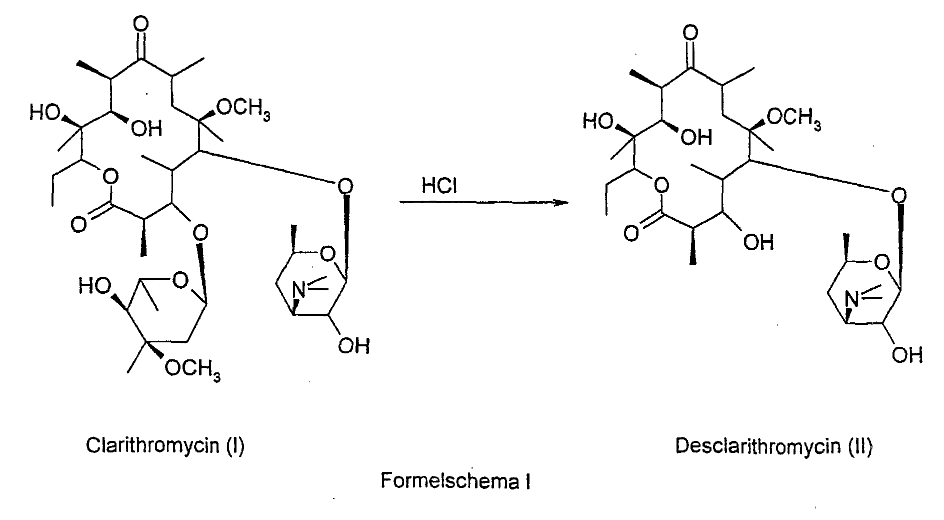

Weiterhin kann Desclarithromycin (II) direkt aus Erythromycin A (III) nach folgender Synthesesequenz hergestellt werden:
Zunächst wird Erythromycin A (III) durch Einwirken von Hydroxylamin oximiert (siehe z. B. Abbott Laboratories WO 97/38000). Vom resultierenden Erythromycinoxim wird durch saure Behandlung die Cladinose abgespalten und extraktiv entfernt (Formelschema II). Man erhält decladinosiertes Erythromycinoxim (IV).

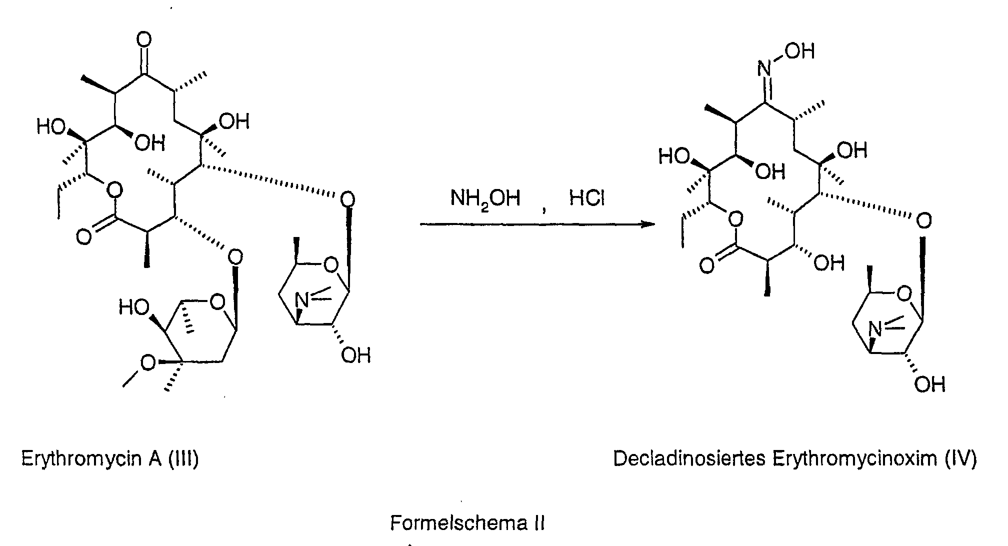

Anschließend werden (gem. Bonnet et. al.United States Patent 5,969,161) die Oximgruppe mit Methoxypropen unter leicht sauren Bedingungen und die Hydroxylgruppen durch Einwirken von Trimethylchlorsilan unter basischen Bedingungen geschützt (Formelschema III).

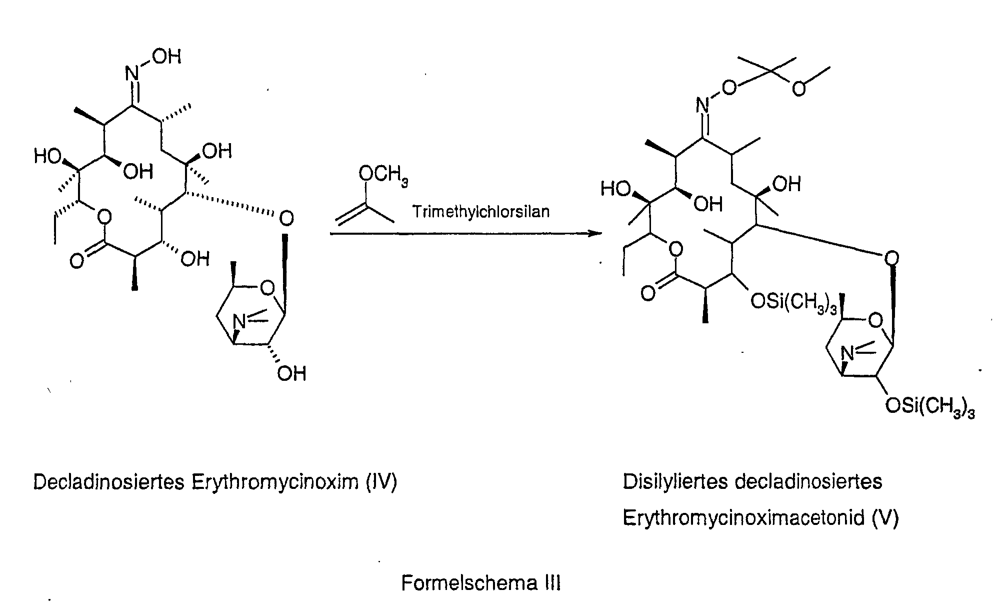

Durch Einwirkung von z. B. Methyljodid und einer starken Base (z. B. Kaliumhydroxid) wird die geschützte Verbindung in 6-O-Stellung methyliert und anschließend durch saure Abspaltung der Schutzgruppen in das Desclarithromycinoxim (VI) überführt (Formelschema IV).

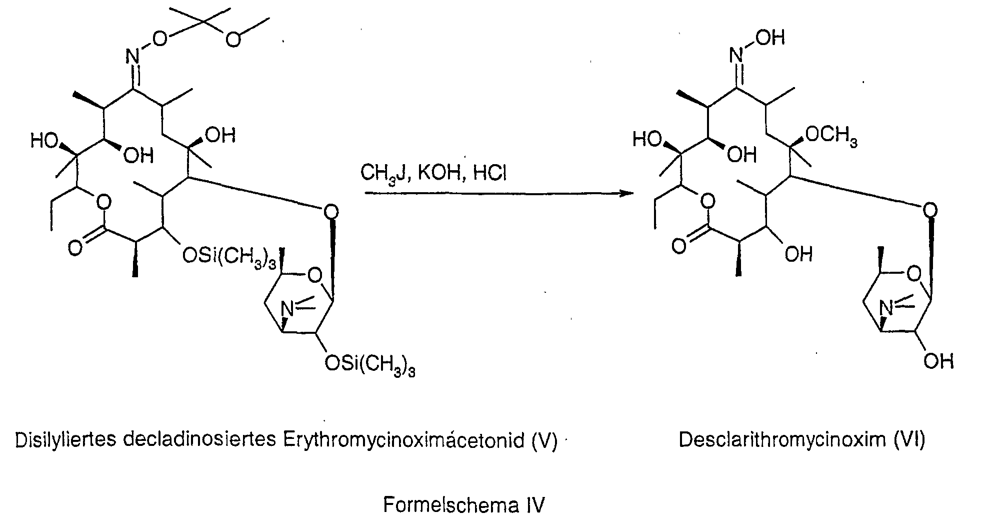

Das Desclarithromycin (II) gewinnt man nun durch Einwirkung von Natriummetabisulfit auf das Desclarithromycin (VI) (Formelschema V).

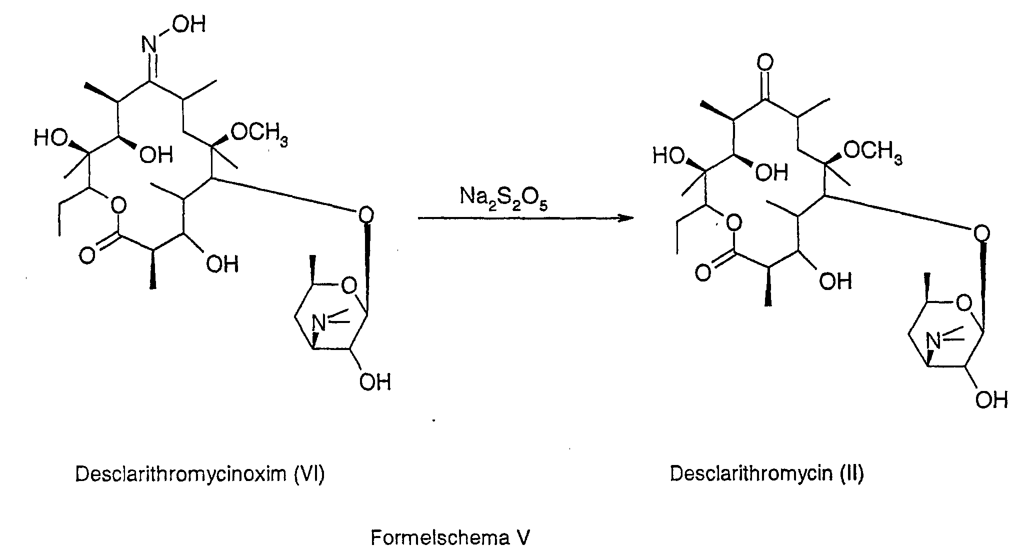

Nachteilig bei diesen Verfahren ist die Entstehung von mehrfach methylierten Nebenprodukten, die neben der Methylierung in 6-O-Stellung noch in der 11- und/oder 12-Stellung des Moleküls methylierte Hydroxylgruppen aufweisen (z. B. Formeln (VII) und (VIII)). Diese stören beim weiteren Verarbeiten des Desclarithromycins (II) zu Makrolidantibiotika und müssen deshalb vorher durch aufwendige Reinigungsprozesse entfernt werden. Weiterhin nachteilig ist die Verwendung von oximierten Zwischenstufen, da hier E-/Z-Isomere auftreten, die unterschiedliche physikalische Eigenschaften besitzen (z. B. Löslichkeit) und deshalb bei der Umarbeitung zu Ausbeuteverlusten führen.

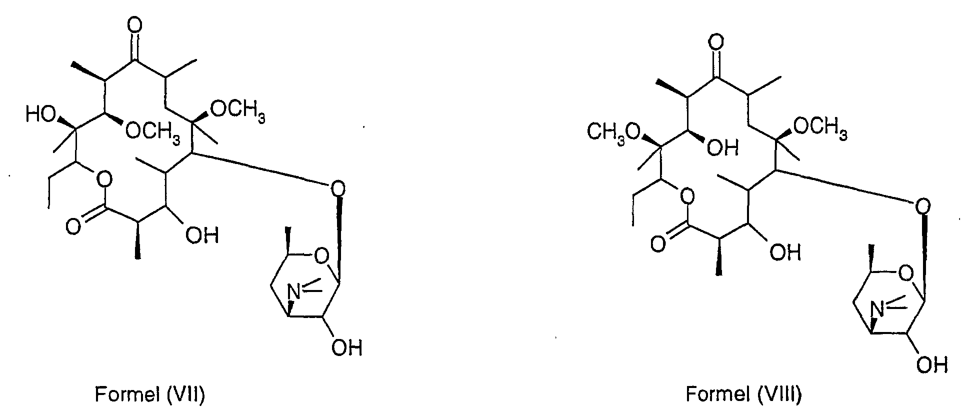

Aufgabe der vorliegenden Erfindung ist es, ein Verfahren zur Herstellung von Desclarithromycin (II) zu finden, das die vorher beschriebenen Nachteile vermeidet. Dies kann erreicht werden, indem man den folgenden Syntheseweg, der durch neuartige Zwischenverbindungen gekennzeichnet ist, einschlägt:

Gegenstand der vorliegenden Erfindung ist ein Verfahren zur Herstellung von Desclarithromycin bei dem Erythromycin A (III) zunächst durch Silylierung mit basischen Mitteln, bevorzugt sind Trialkylchlorsilan und Trialkylsilylimidazol ) in bekannter Weise Y. Kawashima et al, Chem. Pharm. Bull. 38, 1485-1489, 1990 in der 2'-O-Stellung und 4"-O-Stellung silyliert wird (IX). Bevorzugt sind die in Y. Kawashima et al, Chem. Pharm. Bull. 38, 1485-1489, 1990 beschriebenen Bedingungen (Formelschema VI).

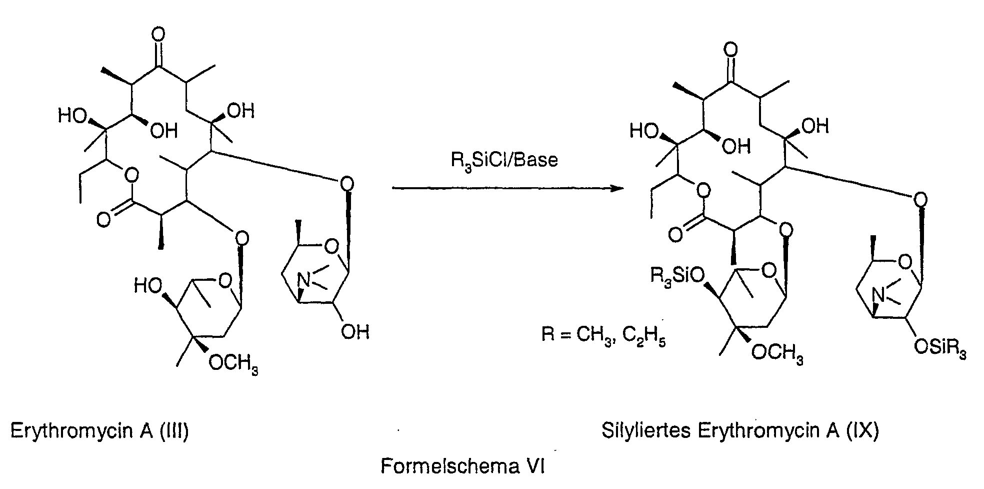

Anschließend wird das silylierte Erythromycin A durch Oxidation mit üblichen Oxidationsmitteln, bevorzugt sind Wasserstoffperoxid oder m-Chlorperbenzoesäure, in das silylierte Erythromycin A N-oxid (X) überführt (Formelschema VII).

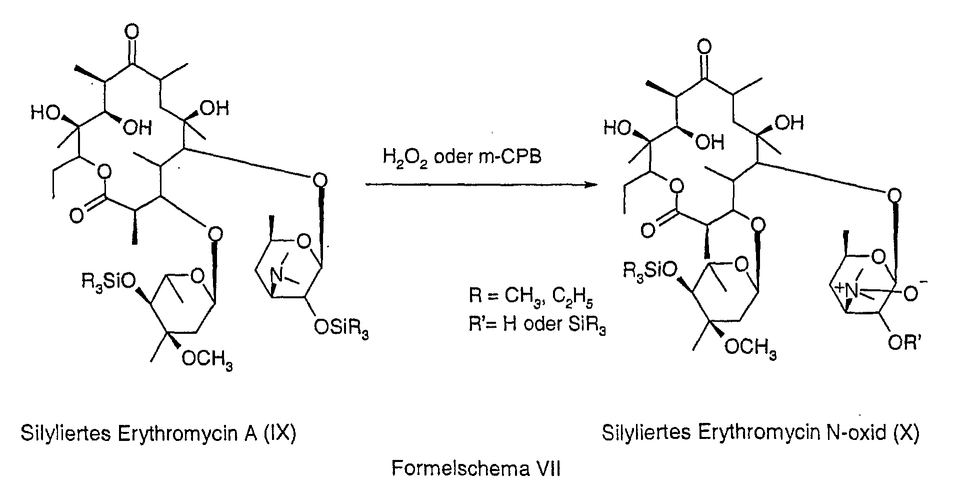

Optional kann man die beiden ersten Schritte auch in der Reihenfolge tauschen (also zunächst das Erythromycin N-oxidieren und dann die Silylschutzgruppen einführen) oder man kann die beiden Schritte in einer Eintopfreaktion verknüpfen.

Danach wird die geschützte Verbindung in 6-O-Stellung mit einem Methylierungsmittel, bevorzugt sind Methyljodid oder Dimethylsulfat, unter basischen Bedingungen, bevorzugt ist die Basischstellung durch Zugabe von Kaliumhydroxid, selektiv methyliert (Formelschema VIII).

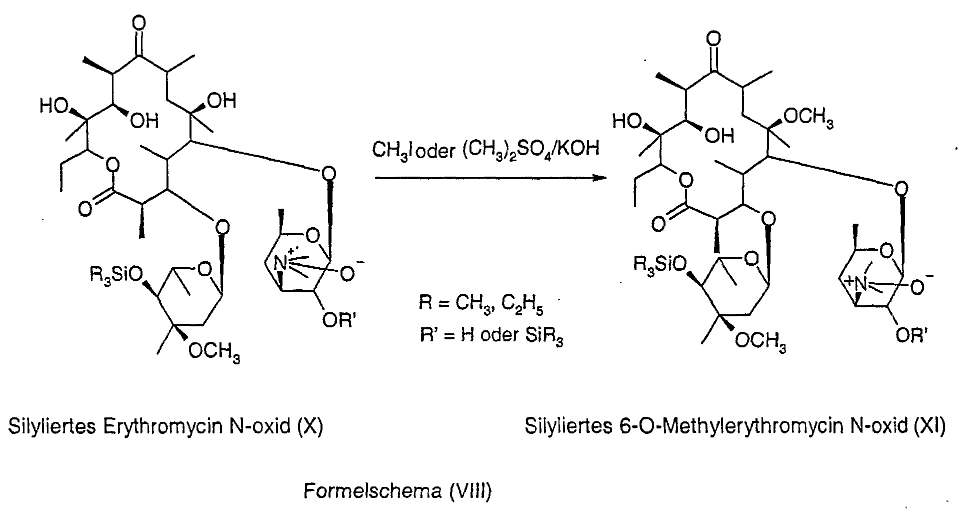

Vom silylierten 6-O-Methylerythromycin N-oxid (XI) werden durch saure Behandlung, bevorzugt ist die Zugabe von HCl, die Cladinose und die Silylschutzgruppen abgespalten (Formelschema (IX). Dadurch erhält man das Desclarithromycin N-oxid (XII).

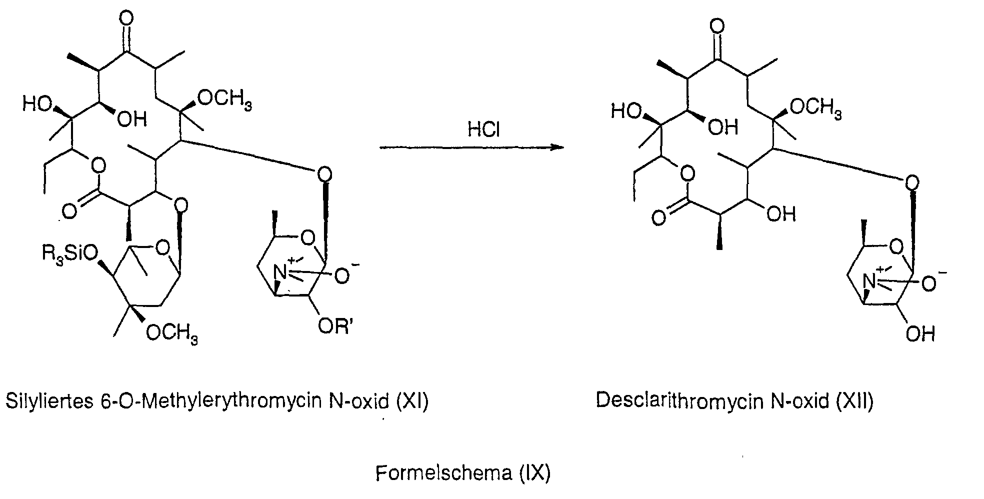

Desclarithromycin N-oxid (XII) wird dann nach bekannten Methoden, vorzugsweise katalytisch mit Palladium/Kohle in Gegenwart von Wasserstoff oder eines Wasserstoffüberträgers,vorzugsweise mit Cyclohexen), Raney Nickel und Wasserstoff oder Natriumbisulfit, zum Desclarithromycin (II) reduziert (Formelschema X).

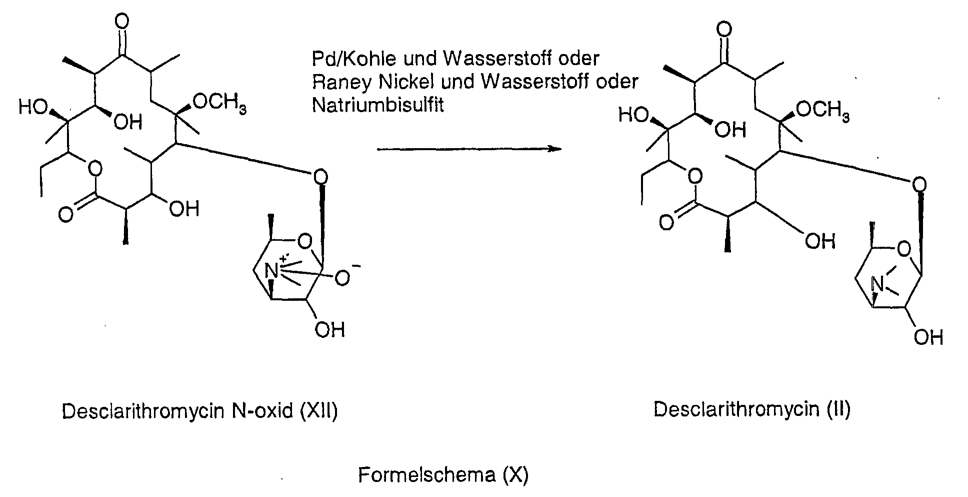

Optional kann man die beiden letzten Schritte (Formelschemata IX und X) auch in der Reihenfolge tauschen (also zunächst das N-Oxid zum Amin reduzieren und dann die Cladinose und die Schutzgruppen abspalten) oder man kann die beiden Schritte in einer Eintopfreaktion verknüpfen, z. B. indem man die gem. Formelschema (IX) erhaltene Lösung von Desclarithromycin N-oxid (XII) mit einer wässrigen Natriumbisulfitlösung verrührt, bis (XII) zu Desclarithromycin (II) reduziert ist und letzteres aus der Reaktionsmischung z.B. durch Kristallisation isoliert.

Die beschriebenen Reaktionsschritte, die durch die Formelschemata (VI) bis (X) erläutert werden, können unter verschiedenen Bedingungen ablaufen. Es ist sinnvoll, die Reaktionsbedingungen unter Berücksichtigung allgemein bekannter einschlägiger Methoden des Standes der Technik zu variieren, um die beste Ausführungsform herauszufinden. Nach derzeitigem Kenntnisstand sind die folgenden Reaktionsbedingungen bevorzugt:

Die in Formelschema VI dargestellte Reaktion findet in einem organischen Lösungsmittel statt, vorzugsweise Ethylacetat, Butylacetat, Dichlormethan, MTB-Ether, THF, Toluol, insbesondere Ethylacetat. Die Reaktion kann bei unterschiedlichen Temperaturen stattfinden, bevorzugt ist die Durchführung bei Raumtemperatur.

Die in Formelschema VII dargestellte Reaktion findet in einem organischen Lösungsmittel statt, vorzugsweise Dichlormethan, Ethylacetat, Butylacetat, DMF, N,N-Dimethylacetamid, NMP, insbesondere Dichlormethan. Die Reaktion kann bei unterschiedlichen Temperaturen stattfinden, bevorzugt ist die Durchführung bei ca. 0° C.

Die in Formelschema VIII dargestellte Reaktion findet in einem organischen Lösungsmittel statt, vorzugsweise Dimethylsulfoxid, Tetrahydrofuran, DMF, N,N-Dimethylacetamid, NMP, Dimethyltetrahydropyrimidinon (DMPU), insbesondere in einer Mischung aus vorzugsweise gleichen Teilen Dimethylsulfoxid und Tetrahydrofuran. Die Reaktion kann bei unterschiedlichen Temperaturen stattfinden, bevorzugt ist die Durchführung bei Raumtemperatur.

Die in Formelschema IX dargestellte Reaktion findet vorzugsweise in wässriger Phase statt. Die Reaktion kann bei unterschiedlichen Temperaturen stattfinden, bevorzugt ist die Durchführung bei Raumtemperatur.

Die in Formelschema X dargestellte Reaktion findet in einem organischen Lösungsmittel statt, vorzugsweise Dichlormethan, Ethylacetat, Butylacetat, THF, DMF, N,N-Dimethylacetamid, NMP, insbesondere Dichlormethan. Die Reaktion kann bei unterschiedlichen Temperaturen stattfinden, bevorzugt ist die Durchführung bei Raumtemperatur.

Für die Reaktionen gemäß der Formelschemata VI bis X kann es hilfreich sein, die Reaktion unter einer Schutzgasatmosphäre durchzuführen. Die Isolation der erhaltenen Produkte kann auf unterschiedliche Weise stattfinden, wie z.B. durch Abfiltrieren, Extrahieren, durch Chromatographie etc..

Die Verbindungen der Formeln X, XI und XII sind bisher nicht bekannt und sind, wie auch die Verfahren zu ihrer Herstellung, ebenfalls Gegenstand der vorliegenden Erfindung. Die genannten Verbindungen eignen sich insbesondere als Zwischenprodukte in der chemischen Synthese, insbesondere bei der Herstellung von Desclarithromycin.

Durch die nachfolgenden Ausführungsbeispiele soll die vorliegende Erfindung näher erläutert werden, ohne dass die Erfindung auf die in den Beispielen beschriebene Durchführungsform eingeschränkt wird. Die einzelnen Merkmale der Beispiele stehen für spezielle Ausführungsformen, die mit verallgemeinerten Merkmalen wie im Beschreibungstext und/oder in den Ansprüchen offenbart kombiniert werden können.

DC-Analysen wurden auf beschichteten Glasplatten (5 x 20 cm, Kieselgel 60 F₂₅₄ der Firma Merck Darmstadt) im aufsteigenden Modus durchgeführt, wobei die Gasphase mit Eluentendampf gesättigt war. Das Anfärben (Detektion der getrennten Reaktionsprodukte) nach der Entwicklung der DC-Platte und Trocknung per Warmluftfön erfolgte durch kurzes Eintauchen der DC-Platte in eine Lösung von 25 g Molybdatophosphorsäure und 10g Cer(IV)sulfat in 940 ml Wasser und 60 ml konz. Schwefelsäure, Abtropfen und abschließendes Erhitzen der DC-Platte auf ca. 160°C auf einer DESAGA Thermoplate S ™. ¹H- und ¹³C-NMR-Spektrum wurden mit einem Bruker 400 UltraShield™ - Spektrometer gemessen. Für die Interpretation der ¹³C-NMR-Spektren wurden 1°, 2°, 3° und 4° Kohlenstoffatome durch Messung von DEPT 135° - Spektren differenziert. Es wurden aber keine mehrdimensionalen Spektren (¹H-¹H- oder ¹H-¹³C-Korrelation) gemessen. Darum ist nicht auszuschließen, daß, insbesondere bei Protonen oder ¹³C-Atomen gleicher Multiplizität, Signalzuordnungen wechselseitig getauscht werden müssen.

### Beispiel 1:

### Synthese von 2',4"-O-Bis(trimethylsilyl)erythromycin A (Formel IX, R = CH₃) [modifiziert, aufbauend auf Y. Kawashima et al Chem. Pharm. Bull. 38, 1485-1489 (1990)]

Bei diesem Ansatz wurde Erythromycin A der Firma Abbott Laboratories eingesetzt, das 94,0 HPLC-Flächenprozente Erythromycin A enthielt. Der Wassergehalt gemäß Karl-Fischer-Titration betrug 0,5 Gew.-%.

In einem 2L-Kolben mit mechanischem Rührer, Thermometer und Tropftrichter wurden unter Stickstoffatmosphäre 36,7 g (50,0 mmol tel qel, 47,0 mmol Gehalt) Erythromycin A in 1000 ml Ethylacetat klar gelöst. Unter Beibehaltung der Temperatur von 20°C (Wasserbad) tropfte man eine Lösung aus 8,15 g (74,3 mmol) Trimethylchlorsilan und 10,52 g (72,7 mmol) N-(Trimethylsilyl)-imidazol in 50 ml Ethylacetat innerhalb 30 Min. zu. Die Reaktion war exotherm. 15 Min. nach Beginn des Zutropfens bildete sich ein Niederschlag, der sich zunächst zu Klumpen zusammenballte, der aber später zu einer gut verteilten Suspension wurde. DC-Monitoring (CH₂Cl₂ / MeOH 9:1 plus 1% Ammoniaklösung 25%ig) zeigte nach 0,25 Std. vollständigen Umsatz des Erythromycins A (R_{f} = 0,43) zur Titelverbindung (ca. 70%; R_{f} = 0,67) und dem Monosilyl-Produkt (ca. 30%; R_{f} = 0,54) an. Nach 2.5 Std. hatte sich das Monosilyl-Zwischenprodukt bis auf einen Rest von ca. 5% in die Titelverbindung umgewandelt. Die Suspension wurde unter magnetischem Rühren zu einer eiskalten Lösung von 15 g (178,6 mmol) Natriumhydrogencarbonat in 285 ml Wasser gegossen. Die wäßrige Phase wurde abgetrennt und die organische Phase wurde erst mit 300 ml Wasser, dann mit 300 ml gesättigter Kochsalzlösung gewaschen. Die organische Phase wurde über Magnesiumsulfat getrocknet, filtriert, bei 40°C Badtemperatur im Vak. zur Trockne eingeengt und der kristalline Rückstand im Hochvakuum (HV) getrocknet (44.6 g, 101,5% d.Th. Rohprodukt). Der Rückstand wurde mit 150 ml n-Heptan versetzt und langsam erwärmt. Bei 84°C bildete sich eine klare farblose Lösung. Man ließ unter Entfernung des Heizbads abkühlen und impfte bei 65°C mit Kristallen der Titelverbindung an. Unter mechanischem Rühren (320 U/min) ließ man weiter auf Raumtemperatur abkühlen, kühlte dann auf 15°C und rührte weitere 15 Min. bei dieser Temperatur. Der Niederschlag wurde über eine Glasfritte G4 abgesaugt, mit 20 ml n-Heptan nachgewaschen, dann im Vakuum im Stickstoffstrom bei 40°C getrocknet. Man erhielt 31,0 g weiße Kristalle. Die Mutterlauge wurde im schwachen Vak. auf ca. ein Drittel des Volumens eingeengt, wobei sich die Lösung leicht trübte. Es wurde auf 10°C abgekühlt und 15 Min. bei dieser Temperatur nachgerührt. Der Niederschlag wurde abgesaugt, mit 10 ml n-Heptan nachgewaschen, dann im HV getrocknet. Man erhielt 4,4 g weiße Kristalle. Gesamtausbeute: 35,4 g (40.3 mmol, 85,7% d.Th.), Schmp. 213-215°C (Lit. 194-197°C). ¹H-NMR (400 MHz, CDCl₃): δ = 4,98 (dd, 1H, 13-H), 4,83 (d, 1H, 1"-H), 4,39 (d, 1H, 1'-H), 4,22 (m, 1H, 5"-H), 4,16 (d, 1H, 3-H), 3,83 (1H, 11-OH), 3,80 (1H, 11-H), 3,59 (m, 1H, 5'-H), 3,56 (d, 1H, 5-H), 3,30 (s, 3H, 3"-OMe), 3,18 (m, 1H, 2'-H), 3,17 (d, 1H, 4"-H), 3,11 (qua, 1H, 10-H), 3,01 (s, 1H, 12-OH), 2,80 (qui, 1H, 2-H), 2,74 (m, 1H, 8-H), 2,53 (m, 1H, 3'-H), 2,37 (d, 1H, 2"-H), 2,23 (br s, 6H, NMe₂), 1,97-1,82 (m, 3H, 14-H, 4-H, 7-H), 1,72-1,60 (m, 4H, 7-H, 6-OH, 4'-H), 1,55-1,45 (m, 2H, 2'-H, 14-H), 1,44 (s, 3H, 6-Me), 1,23-1,10 (22H, 6"-Me, 8-Me, 3"-Me, 4'-H, 6'-Me, 12-Me, 10-Me, 2-Me), 1,09 (d, 3H, 4-Me), 0,87 (t, 3H, 15-H), 0,16 (s, 9H, 4"-OSiMe₃), 0,10 (s, 9H, 2'-OSiMe₃). ¹³C-NMR (100 MHz, CDCl₃): δ = 221,3 (C-9), 176,4 (C-1), 102,9 (C-1'), 96,8 (C-1 "), 81,7 (C-5), 81,0 (C-4"), 79,6 (C-3), 77,1 (C-13), 75,3 (C-6), 75,0 (C-12), 73,3 (C-2'), 73,1 (C-3"), 69,0 (C-11), 67,8 (C-5'), 65,2 (C-3'), 65,1 (C-5"), 49,8 (3"-OMe), 44,9 (C-2), 44,4 (C-8), 41,0 (NMe₂), 40,5 (C-4), 39,0 (C-7), 38,7 (C-10), 35,9 (C-2"), 29,8 (C-4'), 27,3 (6-Me), 22,2 (5'-Me), 21,7 (3"-Me), 21,4 (C-14), 19,4 (5"-Me), 18,3 (8-Me), 16,4 (12-Me), 15,6 (2-Me), 11,8 (10-Me), 10,9 (C-15), 9,7 (4-Me), 1,02 [2'-OSi(CH₃)₃], 0,96 [4"-OSi(CH₃)₃]. MS (ESI): [M+H]⁺ m/z = 878 (C₄₃H₈₃NO₁₃Si₂).

Anstatt aus n-Heptan gelingt Umkristallisation / Umfällung des Produkts auch mit hoher Ausbeute und Reinheit aus Aceton-Wasser.

### Beispiel 2:

### Synthese von 4"-O-(Trimethylsilyl)erythromycin A N-oxid (Formel X, R =CH₃, R' =H)

a) Herstellung von 99%iger 3-Chlor-peroxybenzoesäure aus kommerzieller 77% iger 3-Chlor-peroxybenzoesäure (Aldrich):
   In einem 1 L-Rundkolben mit KPG-Rührer, Thermometer und kalibrierter pH-Elektrode wurden unter Stickstoffatmosphäre 400 ml Phosphatpuffer pH 7 (Riedel 10240) vorgelegt. Durch Zugabe von insgesamt 8,47 g Di-Natriumhydrogenphosphat wurde ein pH von 7,5 eingestellt. Dazu gab man auf einmal 50,16 (223,8 mmol) 77%ige 3-Chlor-peroxybenzoesäure. Es bildete sich eine Suspension. Der pH fiel zunächst rasch und dann langsamer, bis er bei pH 6,42 zum Stillstand kam. Durch Zugabe von 13,92 g Di-Natriumhydrogenphosphat wurde der pH wieder auf 6,95 angehoben. Die Suspension wurde abgesaugt. Der Feststoff wurde mit Wasser, das zuvor auf pH 7 eingestellt worden war, gewaschen und anschließend im Exsiccator im HV getrocknet. Man erhielt 31,9 g weiße Kristalle (83% d.Th. bez. auf den Gehalt des eingesetzten kommerziellen Materials). Der Wassergehalt (K.F.-Titr.) betrug 0,27%.
b) 4"-O-(Trimethylsilyl)erythromycin A N-oxid:
   In einem 250ml-Kolben mit mechanischem Rührer, Thermometer und Tropftrichter wurden unter einer Stickstoffatmosphäre 13,18 g (15,0 mmol) Disilylerythromycin A (aus Beispiel 1) in 25 ml Dichlormethan (0,025% Wassergehalt gemäß K.-F.-Titrat.) klar gelöst und 2,27 g (27,0 mmol) trockenes Natriumhydrogencarbonat zugegeben. Die Suspension wurde mit einem Eisbad auf 0°C gekühlt. Dazu tropfte man eine Lösung aus 3,12 g (17.9 mmol) der obigen, ca. 99%igen 3-Chlor-peroxybenzoesäure in 50 ml Dichlormethan. Man entfernte das Kältebad, ließ auf 23°C erwärmen und rührte 1 Std. bei dieser Temperatur nach, wobei sich eine dicke Suspension bildete. DC (CH₂Cl₂ / MeOH 9:1 plus 1% Ammoniaklösung 25%ig) einer abgesaugten Probe des Niederschlags zeigte saubere quantitative Umwandlung des Edukts (R_{f} = 0,67) zum Produkt (R_{f} = 0.25) an. Die Suspension wurde im Eisbad auf 2°C abgekühlt, mit 40 ml halbgesättigter wäßriger Natriumhydrogencarbonatlösung versetzt und intensiv gerührt. Das Gemisch wurde abgesaugt und mit 2 x 10 ml gekühlter halbgesättigter Natriumhydrogencarbonatlösung nachgewaschen, scharf abgesaugt und dann im HV über Phosphorpentoxid getrocknet. Man erhielt 11,8 g (14,4 mmol, 95,6% d.Th.) farblose Kristalle, Schmp. 204 - 205°C (Zersetzung). ¹H-NMR (400 MHz, CDCl₃): δ = 5,03 (dd, 1H, 13-H), 4,89 (d, 1H, 1"-H), 4,68 (d, 1H, 1'-H), 4,18 (m, 1H, 5"-H), 3,98 (d, 1H, 3-H), 3,88 (s, 1H, 11-OH), 3,84 (m, 1H, 5'-H), 3,80 (m, 1H, 11-H), 3,74 (dd, 1H, 2'-H); 3,58 (d, 1H, 5-H), 3,47 (m, 1H, 3'-H), 3,36 (s, 3H, 3"-OMe), 3,18 und 3,17 (2 x s, 2 x 3H, N(O)Me₂), 3,16 (verdeckt, 1H, 4"-H), 3,09 (qua, 1H, 10-H), 3,05 (s, 1H, 12-OH), 2,90 (qui, 1H, 2-H), 2,68 (m, 1H, 8-H), 2,38 (d, 1H, 2"-H), 2,36 (s, 1H, 6-OH), 2,06-1,83 (m, 4H, 4'-, 4-, 7-, 14-H), 1,71 (d, 1H, 2'-OH), 1,57 - 1,45 (m, 3H, 4'- 2"-, 14-H), 1,45 (s, 3H, 6-Me), 1,30 (m, 1H, 7-H), 1,24 - 1,08 (24H, 8 x Me), 0,85 (t, 3H, 15-H), 0,15 (s, 9H, 4"-OSiMe₃). ¹³C-NMR (100 MHz, CDCl₃): δ = 221,7 (C-9), 175,9 (C-1), 101,8 (C-1'), 96,2 (C-1"), 83,1 (C-5), 80,7 (C-3), 79,3 (C-4"), 76,6 (C-13), 75,8 (C-3'), 74,8 und 74,7 (C-6, C-12), 73,2 (C-3"), 72,8 (C-2'), 68,9 (C-11), 66,1 (C-5'); 65,0 (C-5"), 58,8 [N(O)-CH₃], 51,8 [N(O)-CH₃], 49,7 (3"-OCH₃), 45,1 (C-2), 44,6 (C-8), 39,3 (C-4), 38,5 (C-7), 37,8 (C-10), 35,6 und 35,0 (C-2", C-4'), 26,8 (6-CH₃), 22,2 (5'-CH₃), 21,6 (3"-CH₃), 21,1 (C-14), 19,3 (5"-CH₃), 18,3 (8-CH₃), 16,1 (12-CH₃), 15,9 (2-CH₃), 12,0 (10-CH₃), 10,6 (C-15), 9,1 (4-CH₃), 0,9 [4"-OSi(CH₃)₃]. MS (ESI) m/z = 822 (C₄₀H₇₅NO₁₄Si).

Setzt man 1,0 Äquiv. Disilyl-Erythromycin (Beispiel 1) in Dichlormethan bei 0°C mit 1,25 Äquiv. kommerzieller 77%iger 3-Chlorperoxybenzoesäure um (Bildung eines Zweiphasengemischs), so erfolgt ebenfalls saubere Bildung des N-Oxids, welches in einer Ausbeute von 90-93% d.Th. isoliert werden kann.

### Beispiel 3:

### Methylierung von 4"-O-(Trimethylsilyl)-erythromycin A N-oxid zu 4"-O-(Trimethylsilyl)-clarithromycin N-oxid [6-O-Methyl-4"-O-(trimethylsilyl)-erythromycin A N-oxid ; Formel XI, R = CH₃, R' = H]

In einem 100 ml-Kolben mit mechanischem Rührer, Thermometer und Septum wurden unter Stickstoffatmosphäre 5,48 g (6,66 mmol) 4"-O-(Trimethylsilyl) erythromycin A N-oxid (aus Beispiel 2) in 25 ml Dimethylsulfoxid und 25 ml Tetrahydrofuran zu einer dünnen Suspension verrührt. Mit einem Eisbad wurde auf 0°C gekühlt. 559 mg (8,47 mmol) 85%iges Kaliumhydroxid-Pulver wurden auf einmal zugegeben. Es bildete sich eine kräftig gelbe, trübe Lösung, der unter Rühren bei 0°C 1,04 ml (16,40 mmol) Methyliodid zugesetzt wurden, wobei die Reaktionstemperatur von +2 auf +4°C anstieg. Innerhalb einer halben Stunde ließ man das Reaktionsgemisch unter Rühren auf Raumtemperatur erwärmen, wobei es blaßgelb wurde. Man rührte noch 2 Stunden bei Raumtemperatur und gab dann 180 ml Ethylacetat und 120 ml Eiswasser zu. Die wäßrige Phase wurde abgetrennt und die organische Phase mit 120 ml Wasser und dann mit 50 ml Wasser gewaschen. Die vereinigten wäßrigen Waschphasen wurden sofort mit 50 ml Ethylacetat zurückextrahiert und dieses Extrakt mit 20 ml Wasser / 5 ml gesättigter Kochsalz-lösung gewaschen. Die vereinigten organischen Phasen wurden über Natriumsulfat getrocknet, filtriert, im Vakuum eingeengt und der Rückstand im HV zum festen Schaum getrocknet. Man erhielt 5,23 g (6,25 mmol, 94% d.Th.) Rohprodukt, das zu ca. 50-60% aus der Titelverbindung bestand. ¹H-NMR (400 MHz, CDCl₃): δ = 3,37 (s, 3H, 3"-OMe), 3,22 [2 x s, 6H, 3'-N(O)Me₂], 3,04 (s, 3H, 6-OMe), 0,15 (s, 9H, 4"-OSiMe₃). ¹³C-NMR (100 MHz, CDCl₃): δ = 221,5 (C-9), 176,2 (C-1), 102,6 (C-1'), 58,6 [N(O)CH₃], 52,5 [N(O)CH₃], 51,0 (6-OCH₃), 49,9 (3"-OCH₃), 0,9 [4"-OSi(CH₃)₃]. MS (ESI): [M+H]⁺ m/z = 836 (C₄₁H₇₇NO₁₄Si).

### Beispiel 4:

### Cladinose- und Silyl-Abspaltung zu Desclarithromycin N-oxid [6-O-Methyl-erythromycin A N-oxid; Formel XII]

In einem 100 ml-Kolben mit mechanischem Rührer gab man zu 5,2 g (6,22 mmol) des Rohprodukts aus Beispiel 3 unter Stickstoffatmosphäre und Eisbadkühlung die Lösung von 3,2 ml 12 N Salzsäure (38,4 mmol HCl) in 32 ml Wasser. Das Reaktionsgemisch wurde 2 Stunden bei Raumtemperatur gerührt, dann mit Kochsalz gesättigt, mit wäßriger Ammoniaklösung auf pH 8 eingestellt und mit 5mal 50 ml Ethylacetat extrahiert. Die vereinigten Extrakte wurden über Natriumsulfat getrocknet, filtriert, im Vakuum zur Trockne eingedampft und im HV getrocknet. Man erhielt 4,7 g blaßbraunen Feststoff.

Eine analytische Probe wurde per Flash-Chromatographie einer Probe (200 mg) dieses Rohprodukts durch 40 g Kieselgel 60 (Merck, 0,040 - 0,063 mm) mit dem Eluenten Dichlormethan / Methanol 8:2 gewonnen. Erhalten wurden 95 mg weiße Kristalle, Schmp. 209 - 210°C (Zersetzung), >97% rein gemäß HPLC-Analyse (LiChroCART 125 x 4 mm, LiChrospher 100 RP18e, 5 µm, Det. 210 nm, 25°C, Fluß 0,5 ml / min, Eluent A: CH₃CN / CF₃CO₂H 1000 : 0,5 , Eluent B: H₂O / CF₃CO₂H 1000 : 0,5 ; linearer Gradient von 30% A / 70% B bei Injektion zu 50% A / 50% B nach 10 Minuten Chromatographiedauer; Retentionszeit: 8,55 min), einheitlicher Fleck gemäß DC-Analyse (CH₂Cl₂ / CH₃OH 8:2 plus 1% Ammoniaklösung 25%ig , R_{f} = 0,34). ¹H-NMR (400 MHz, CDCl₃): δ = 5,17 (dd, 1H, 13-H), 4,48 (d, 1H, 1'-H), 3,88 (s, 1H, 11-OH), 3,86 (d, 1H, 11-H), 3,80 (dd, 1H, 2'-H), 3,72 (s, 1H, 5-H), 3,64 (m, 1H, 5'-H), 3,57 (d, 1H, 3-H), 3,38 (m, 1H, 3'-H), 3.27 (s, 1H, 12-OH), 3,18 [s, 3H, N(O)Me], 3,15 [s, 3H, N(O)Me], 3,01 (m, 1H, 10-H), 2,97 (s, 3H, 6-OMe), 2,65 (m, 1H, 2-H), 2,57 (m, 1H, 8-H), 2,09 (qua, 1H, 4'-H), 2,02 - 1,87 (m, 3H, 4-, 7-, 14-H), 1,53 (d, 1H, 2'-OH), 1,49 (m, 1H, 14-H), 1,40 (d, 1H, 4'-H), 1,37 (s, 3H, 12-Me), 1,31 (d, 3H, 5'-Me), 1,27 (d, 3H, 2-Me), ca. 1.26 (m, verdeckt, 1H, 7-H), 1,19 (s, 3H, 6-Me), 1,16 (d, 3H, 8-Me), 1,15 (d, 3H, 10-Me), 1,13 (d, 3H, 4-Me), 0,84 (t, 3H, 15-H). ¹³C-NMR (100 MHz, CDCl₃): δ = 220,4 (C-9), 175,2 (C-1), 106,1 (C-1'), 89,0 (C-5), 78,7 (C-3), 78,0 (C-6), 76,5 (C-13), 75,7 (C-3'), 74,2 (C-12), 72,1 (C-2'), 69,7 (C-5'), 68,2 (C-11), 59,0 [N(O)-CH₃], 51,9 [N(O)-CH₃], 49,4 (6-OCH₃), 45,4 (C-2), 44,6 (C-8), 38,7 (C-7), 37,6 (C-4), 36,0 (C-10), 34,4 (C-4'), 21,4 (C-14), 20,9 (5'-CH₃), 18,7 (6-CH₃), 17,7 (8-CH₃), 16,2 (12-CH₃), 15,3 (2-CH₃), 12,5 (10-CH₃), 10,3 (C-15), 8,3 (4-CH₃). MS (ESI): [M+H]⁺ m/z = 606 (C₃₀H₅₅NO₁₁).

### Beispiel 5:

### Reduktion des rohen N-oxids zu Desclarithromycin [6-O-Methyl-erythromycin A; Formel II]

In einem 100 ml-Kolben mit mechanischem Rührer wurden 4,5 g des rohen Desclarithromycin-N-oxids aus Beispiel 4 mit 50 ml Dichlormethan und der Lösung von 1,5 g (7,9 mmol) Natriummetabisulfit (Na₂S₂O₅) in 15 ml Wasser versetzt und das Zweiphasengemisch wurde unter Stickstoffatmosphäre heftig bei Raumtemperatur gerührt. Die Reduktion konnte mit dem in Beispiel 4 beschriebenen HPLC-System (Retentionszeit von II = 7,37 min) und mit dem in Beispiel 4 beschriebenen DC-System (R_{f} II = 0,52) verfolgt werden und war nach 3 Stunden vollständig. Die wäßrige Phase wurde abgetrennt und mit 20 ml Dichlormethan extrahiert. Die vereinigten organischen Phasen wurden im Vakuum auf ca. 15 ml eingeengt, dann mit 45 ml Wasser versetzt und mit 36%iger Salzsäure pH 1,0 eingestellt. Die organische Phase wurde abgetrennt und restliche Cladinose und deren Folgeprodukt durch Extraktion mit 5 x 10 ml Dichlormethan aus der sauren wäßrigen Phase ausgewaschen. Dieser Vorgang konnte mit dem in Beispiel 4 beschriebenen DC-System verfolgt werden. Die wäßrige Phase wurde dann mit 25%igem wäßrigem Ammoniak auf pH 5,3 eingestellt, der Rührer abgestellt, die wäßrige Lösung mit 5 ml Methylisobutylketon (MIBK) überschichtet und das Zweiphasengemisch mit sehr langsamer Rührgeschwindigkeit (ca. 20 U/Min) ohne nennenswerte Phasendurchmischung 15 Minuten bei 20°C gerührt. Die MIBK-Phase (Verunreinigungen enthaltend) wurde im Scheidetrichter abgetrennt und die wäßrige Phase mit 25%igem wäßrigem Ammoniak unter heftigem Rühren bei 25°C (leichte Kühlung) langsam auf pH 9,5 gestellt, wobei die Lösung bei pH 7,5 mit reinen Produktkristallen angeimpft wurde und das Produkt ab ca. pH 8,3 auskristallisierte. Die Suspension wurde noch 30 Minuten bei 25°C und weitere 30 Minuten bei 15 -20°C nachgerührt. Der Niederschlag wurde abgesaugt, mit 30 ml Wasser gewaschen, trockengesaugt und im HV bei 40°C 16 Stunden getrocknet. Erhalten wurden 1,8 g weiße Kristalle (3,05 mmol, 46% d.Th. bezogen auf das in Beispiel 3 eingesetzte 4"-O-(Trimethylsilyl)-erythromycin N-oxid). Unter Berücksichtigung der in Beispiel 4 zur Gewinnung der analytischen Probe entnommenen 200 mg ergibt sich für die in Beispielen 3 bis 5 beschriebenen Reaktionen eine Gesamtausbeute von 48% d.Th.), Schmp. 154 - 155°C. ¹H-NMR (400 MHz, CDCl₃): δ = 5,18 (dd, 1H, 13-H), 4,38 (d, 1H, 1'-H), 3,92 (s, 1H, 11-OH), 3,87 (br s, 1H, 3-OH), 3,86 (d, 1H, 11-H), 3,68 (s, 1H, 5-H), 3,55 (m, 2H, 3- und 5'-H), 3,26 (s, 1H, 12-OH), 3,24 (dd, 1H, 2'-H), 3,01 (qua, 1H, 10-H), 2,97 (s, 3H, 6-OMe), 2,66 (m, 1H, 2-H), 2,58 (m, 1H, 8-H), 2,47 (m, 1H, 3'-H), 2,26 (s, 6H, NMe₂), 2,12 (m, 1H, 4'-H), 1,94 (m, 2H, 4- und 7-H), 1,66 (d qua, 1H, 14-H), 1,56 (dd, 1H, 4'-H), 1,49 (m, 1H, 14-H), 1,37 (s, 3H, 12-Me), 1,26 (d, 6H, 5'-Me und 2-Me), ca. 1,25 (m, verdeckt, 1H, 7-H), 1,18 (s, 3H, 6-Me), 1,13 (d, 6H, 8-Me und 10-Me), 1,12 (d, 3H, 4-Me), 0,84 (t, 3H, 15-H). ¹³C-NMR (100 MHz, CDCl₃): δ = 220,6 (C-9), 175,0 (C-1), 106,6 (C-1'), 88,2 (C-5), 78,9 (C-3), 78,1 (C-6), 76,6 (C-13), 74,2 (C-12), 70,7 (C-2'), 70,2 (C-11), 69,8 (C-5'), 65,6 (C-3'), 49,5 (6-OCH₃), 45,5 (C-2), 44,5 (C-8), 40,2 [3'-N(CH₃)₂], 38,7 (C-7), 37,5 (C-4), 35,9 (C-10), 28,1 (C-4'), 21,4 (C-14), 21,2 (5'-CH₃), 18,8 (6-CH₃), 17,7 (8-CH₃), 16,2 (12-CH₃), 15,2 (2-CH₃), 12,6 (10-CH₃), 10,4 (C-15), 8,2 (4-CH₃). MS (ESI): [M+H]⁺ m/z = 590 (C₃₀H₅₅NO₁₀).

Die nachfolgenden Patentansprüche, die zum Inhalt der Beschreibung gehören, tragen zur Offenbarung der Erfindung bei.

## Patentansprüche

1. Verfahren zur Herstellung von Desclarithromycin, **dadurch gekennzeichnet, dass** a) Erythromycin A mit R₃SiCl und/oder R₃Si-imidazol oder (R₃Si)₂NH oder R₃SiO₃SCF₃ mit R bedeutend CH₃, C₂H₅ unter basischen Bedingungen zu Verbindungen der Formel (IX) umgesetzt wird, und b) 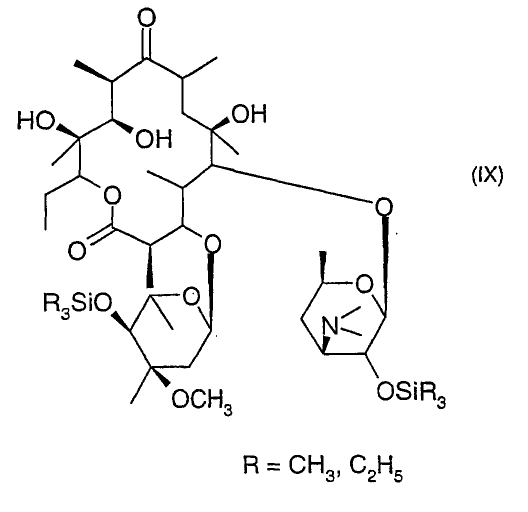 anschließend durch Zugabe eines Oxidationsmittels zu der Verbindung der Formel X oxidiert wird 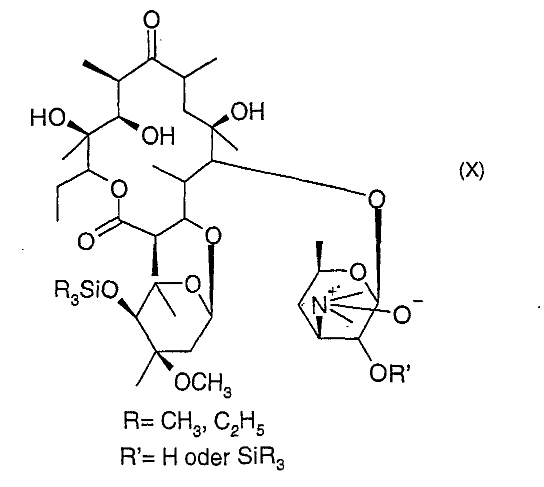 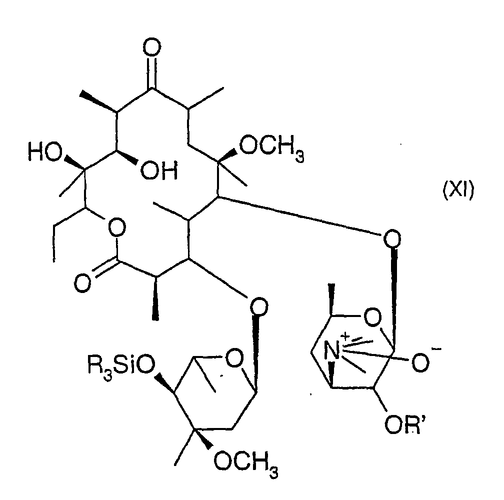 und die erhaltene Verbindung der Formel (X) wird c) durch Zugabe eines Methylierungsmittels unter basischen Bedingungen in die Verbindung der Formel (XI) überführt
und anschließend d) wird die Verbindung der Formel (XI) durch saure Hydrolyse in die Verbindung der Formel (XII) überführt 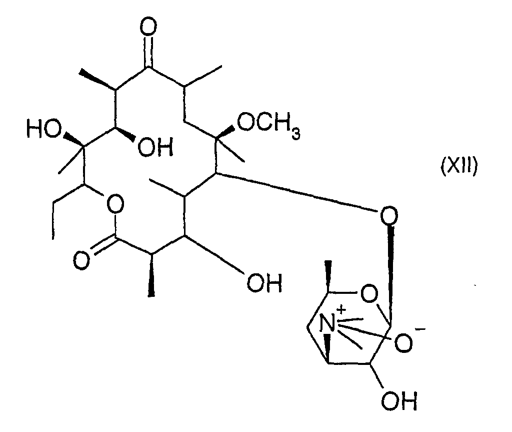 und anschließend e) wird die Verbindung der Formel (XII) unter reduzierenden Bedingungen in Desclarithromycin (II) überführt

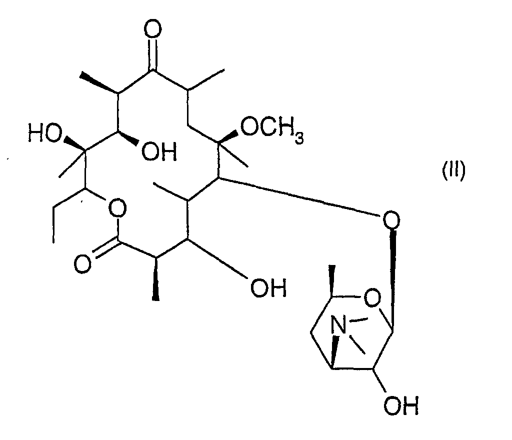

2. Verfahren gemäss Anspruch 1, **dadurch gekennzeichnet, dass** die Abfolge der chemischen Reaktionen der Schritte a) und b) vertauscht wird.

3. Verfahren gemäss den Ansprüchen 1 oder 2, **dadurch gekennzeichnet, dass** die Abfolge der chemischen Reaktionen der Schritte d) und e) vertauscht wird.

4. Verbindungen der Formel (X) 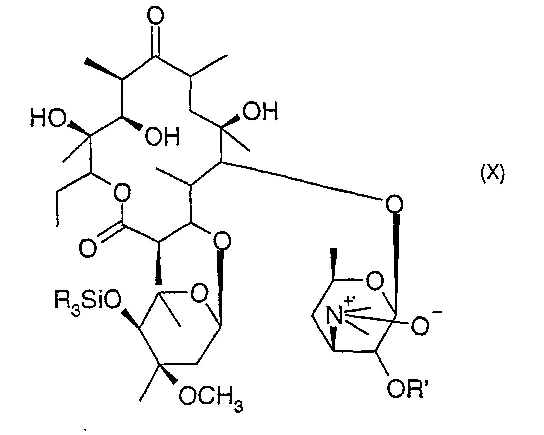 worin R CH₃ oder C₂H₅ bedeutet und worin R' H oder SiR₃ bedeutet.

5. Verfahren zur Herstellung einer Verbindung der Formel (X) gemäss Anspruch 4, **dadurch gekennzeichnet, dass** zu einer Verbindung der Formel (IX) 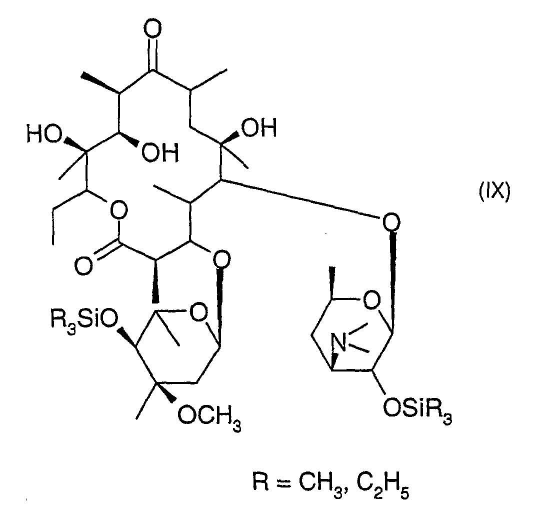 ein Oxidationsmittel hinzugefügt wird
oder dass Erythromycin A durch Zugabe eines Oxidationsmittels oxidiert wird und anschließend eine Umsetzung mit R₃SiCl und/oder R₃Si-imidazol oder (R₃Si)₂NH oder R₃SiO₃SCF₃ mit R bedeutend CH₃, C₂H₅ unter basischen Bedingungen zur Erzeugung der Verbindung der Formel (X) erfolgt.

6. Verbindungen der Formel (XI) 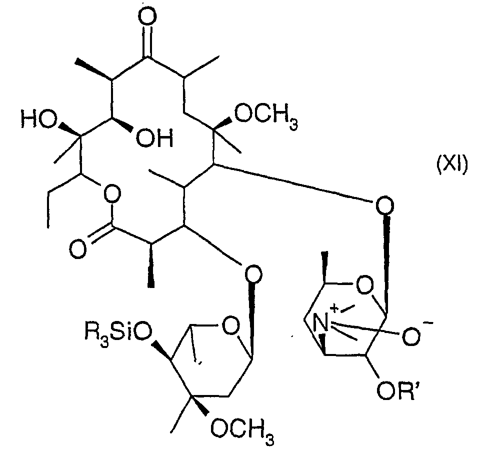 worin R CH₃ oder C₂H₅ bedeutet und worin R' H oder SiR₃ bedeutet.

7. Verfahren zur Herstellung einer Verbindung der Formel (XI) gemäß Anspruch 6, **dadurch gekennzeichnet, dass** eine Verbindung der Formel (X) gemäß Anspruch 4 unter basischen Bedingungen mit einem Methylierungsmittel versetzt wird.

8. Verbindung der Formel (XII)

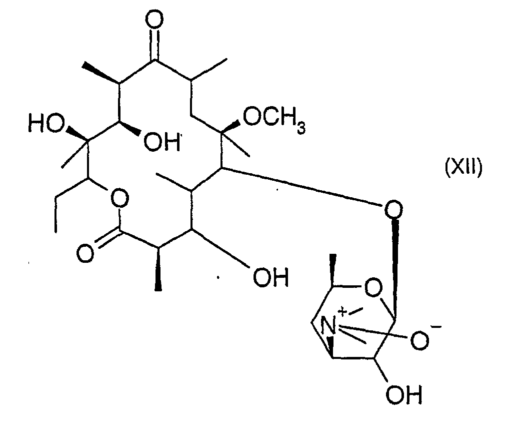

9. Verfahren zur Herstellung der Verbindung der Formel (XII) gemäß Anspruch 8, **dadurch gekennzeichnet, dass** eine Verbindung der Formel (XI) gemäß Anspruch 6 unter sauren Bedingungen hydrolysiert wird.

10. Verwendung von einer oder mehreren der Verbindungen X, XI oder XII gemäß den Ansprüchen 4, 6 oder 8 bei der Herstellung von Desclarithromycin.

## Claims

1. A method for the production of desclarithromycin, which comprises
a) erythromycin A being reacted with R₃SiCl and/or R₃Si-imidazole or (R₃Si)₂NH or R₃SiO₃SCF₃ with R meaning CH₃, C₂H₅ under basic conditions to give compounds of the formula (IX), and b) 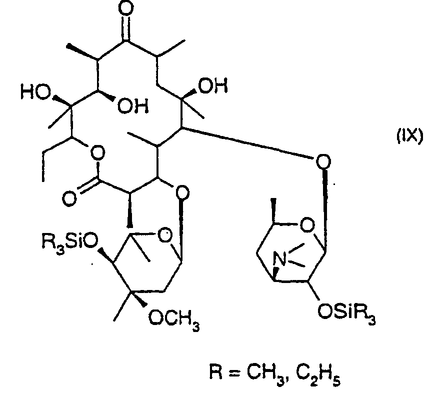 subsequently being oxidized by addition of an oxidizing agent to the compound of the formula X 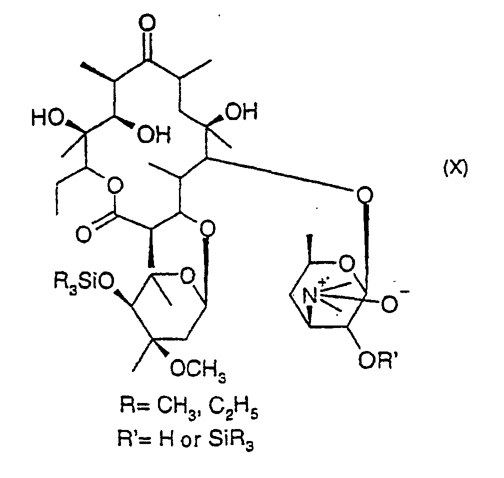 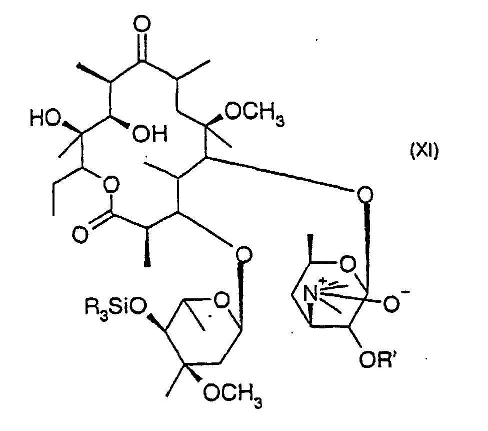 and the resulting compound of the formula (X) being c) converted by addition of a methylating agent under basic conditions into the compound of the formula (XI),
and subsequently d) the compound of the formula (XI) being converted by acid hydrolysis into the compound of the formula (XII) 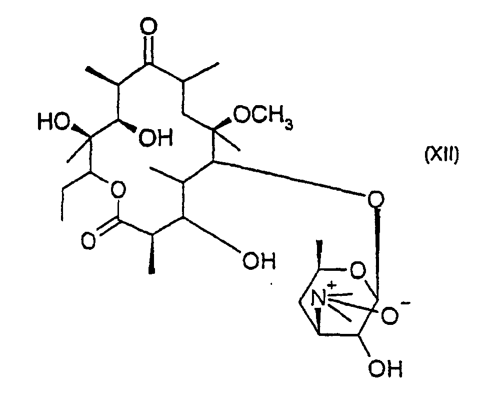 and subsequently e) the compound of the formula (XII) being converted under reducing conditions into desclarithromycin (II)

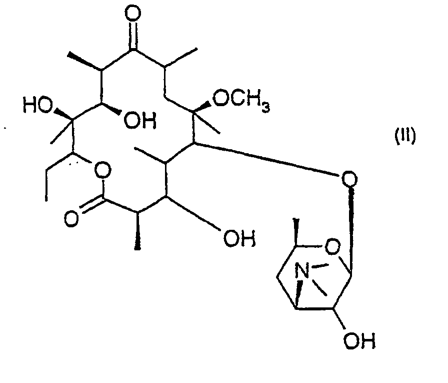

2. The method as claimed in claim 1, wherein the sequence of the chemical reactions of steps a) and b) is changed.

3. The method as claimed in claims 1 or 2, wherein the sequence of the chemical reactions of steps d) and e) is changed.

4. A compound of the formula (X) 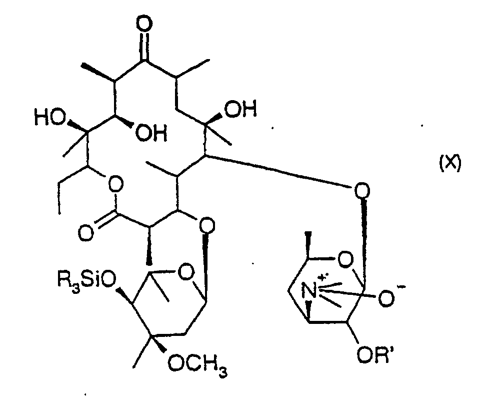 in which R is CH₃ or C₂H₅ and in which R' is H or SiR₃.

5. A method for the production of a compound of the formula (X) as claimed in claim 4, which comprises an oxidizing agent being added to a compound of the formula (IX) 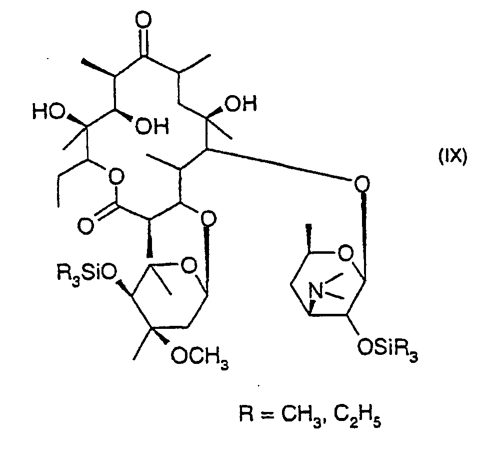 or which comprises erythromycin A being oxidized by addition of an oxidizing agent and subsequently a reaction taking place with R₃SiCl and/or R₃Si-imidazole or (R₃Si)₂NH or R₃SiO₃SCF₃ with R meaning CH₃, C₂H₅ under basic conditions to produce the compound of the formula (X).

6. A compound of the formula (XI) 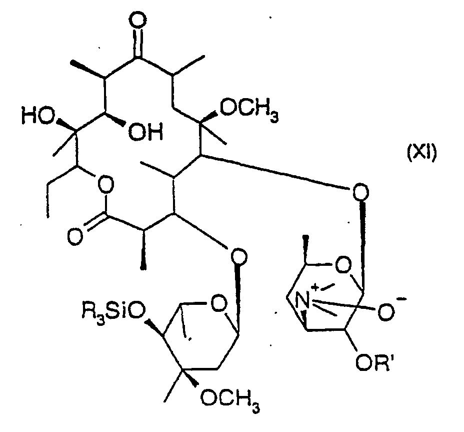 in which R is CH₃ or C₂H₅ and in which R' is H or SiR₃.

7. A method for the production of a compound of the formula (XI) as claimed in claim 6, which comprises a compound of the formula (X) as claimed in claim 4 being mixed with a methylating agent under basic conditions.

8. A compound of the formula (XII)

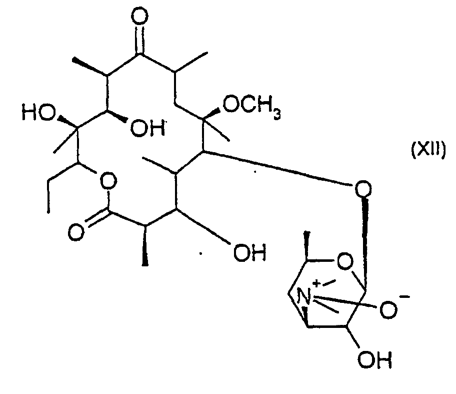

9. A method for the production of the compound of the formula (XII) as claimed in claim 8, which comprises a compound of the formula (XI) as claimed in claim 6 being hydrolyzed under acidic conditions.

10. The use of one or more of the compounds X, XI or XII as claimed in claims 4, 6 or 8 in the production of desclarithromycin.

## Revendications

1. Procédé pour la préparation de desclarithromycine, **caractérisé en ce que**
a) on fait réagir de l'érythromycine A avec R₃SiCl et/ou du R₃Si-imidazole ou (R₃Si)₂NH ou R₃SiO₃SCF₃, où R représente CH₃, C₂H₅, dans des conditions basiques, pour obtenir des composés de formule (IX), 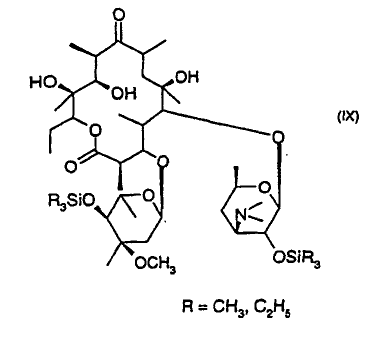
et b) ensuite on les oxyde, par addition d'un oxydant, en le composé de formule X 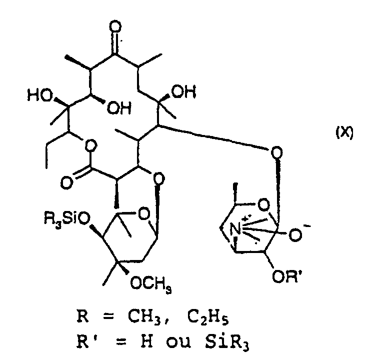
et c) on convertit le composé de formule (X) obtenu, par addition d'un agent de méthylation, dans des conditions basiques, en les composés de formule (XI) 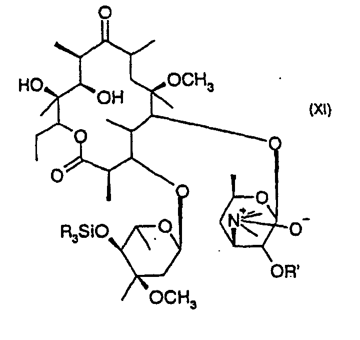 et ensuite d) on convertit le composé de formule (XI), par hydrolyse acide, en le composé de formule (XII) 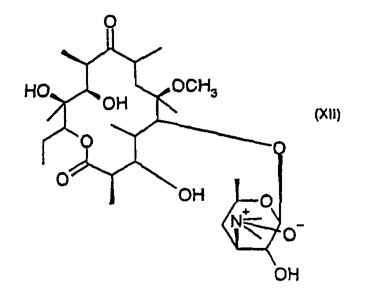 et ensuite e) le composé de formule (XII) est converti, dans des conditions réductrices, en desclarithromycine (II)

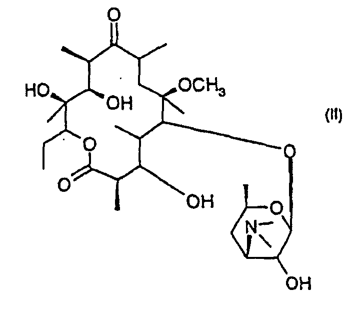

2. Procédé selon la revendication 1, **caractérisé en ce que** l'ordre des réactions chimiques des étapes a) et b) est inversé.

3. Procédé selon la revendication 1 ou 2, **caractérisé en ce que** l'ordre des réactions chimiques des étapes d) et e) est inversé.

4. Composés de formule (X) 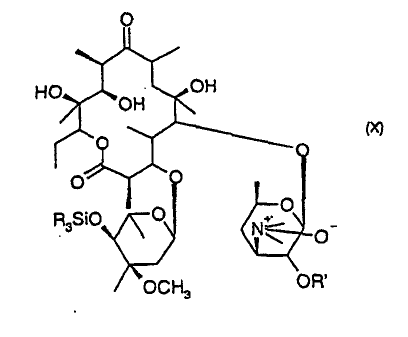 dans laquelle R représente CH₃ ou C₂H₅ et dans laquelle R' représente H ou SiR₃.

5. Procédé pour la préparation d'un composé de formule (X) selon la revendication 4, **caractérisé en ce qu'**on ajoute un oxydant à un composé de formule (IX) 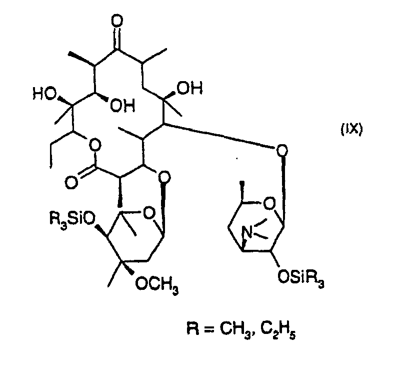 ou **en ce qu'**on oxyde de l'érythromycine A par addition d'un oxydant et on effectue ensuite une réaction avec R₃SiCl et/ou du R₃Si-imidazole ou (R₃Si)₂NH ou R₃SiO₃SCF₃, où R représente CH₃, C₂H₅, dans des conditions basiques, pour l'obtention du composé de formule (X).

6. Composés de formule (XI) 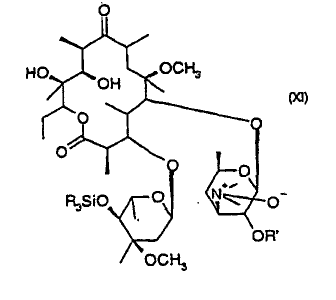 dans laquelle R représente CH₃ ou C₂H₅ et dans laquelle R' représente H ou SiR₃.

7. Procédé pour la préparation d'un composé de formule (XI) selon la revendication 6, **caractérisé en ce qu'**on ajoute un agent de méthylation, dans des conditions basiques, à un composé de formule (X) selon la revendication 4.

8. Composé de formule (XII)

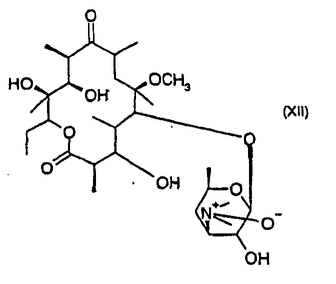

9. Procédé pour la préparation du composé de formule (XII) selon la revendication 8, **caractérisé en ce qu'**un composé de formule (XI) selon la revendication 6 est hydrolysé dans des conditions acides.

10. Utilisation d'un ou plusieurs des composés X, XI ou XII selon les revendications 4, 6 ou 8, dans la préparation de desclarithromycine.
